# EUROPÄISCHE PATENTSCHRIFT

(11) **EP 1 201 718 B1**
(45) Veröffentlichungstag und Bekanntmachung des Hinweises auf die Patenterteilung: **24.05.2006**
(21) Anmeldenummer: 01124502.4
(22) Anmeldetag: 12.10.2001
(51) Int. Cl.: C09B 67/22, C09B 19/02

(54) **Mischkristalle aus Benzimidazolondioxazin-Verbindungen**
Mixed crystals of benzimidazoloneoxazine derivatives
Cristaux mixtes de dérivés de benzimidazolonedioxazine

(30) Priorität: 24.10.2000 DE 10052858
(43) Veröffentlichungstag der Anmeldung: 02.05.2002
(73) Patentinhaber: Clariant Produkte (Deutschland) GmbH, 65929 Frankfurt am Main (DE)
(72) Erfinder: Schmidt, Martin U., Dr., 65931 Frankfurt am Main (DE); Kempter, Peter, Dr., 65812 Bad Soden (DE); Plüg, Carsten, Dr., 79576 Weil am Rhein/Haltingen (DE); Born, Roland, Dr., 68128 Village-Neuf (FR)

(56) Entgegenhaltungen:
- EP-A- 0 321 919
- EP-A- 0 704 497
- EP-A- 0 889 046
- EP-A- 0 911 337
- GB-A- 2 284 427
- US-A- 3 929 719
- US-A- 5 035 747

## Beschreibung

Die vorliegende Erfindung liegt auf dem Gebiet der Dioxazinpigmente, insbesondere der Benzimidazolondioxazine.

Benzimidazolondioxazin-Pigmente sind in DE-A-44 42 291, EP-A-0 911 337, DE-A-197 27 079 und GB-A-22 84 427 beschrieben und zeichnen sich durch rote bis blaue Farbtöne aus.

Der vorliegenden Erfindung lag die Aufgabe zugrunde, Dioxazinpigmente mit hohen Farbstärken, guter Dispergierbarkeit, guter Licht- und Wetterechtheit zur Verfügung zu stellen. Eine weitere Aufgabe war es, Dioxazinpigmente mit besonders rotem Farbton bereitzustellen.

Es wurde gefunden, dass Mischkristalle aus unterschiedlichen Verbindungen der nachstehenden Formel (1) überraschenderweise diese Aufgabe lösen.

Gegenstand der Erfindung sind Mischkristalle aus zwei oder mehreren, vorzugsweise 2, 3 oder 4, voneinander verschiedenen Benzimidazolondioxazin-Verbindungen der allgemeinen Formel (1) 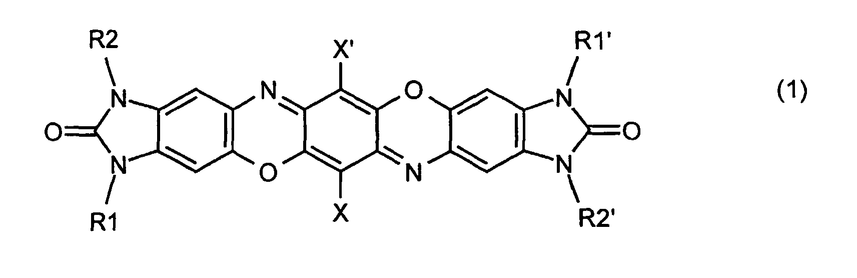 wobei
X und X' gleich sind und für Wasserstoff oder Halogen stehen, R1, R1', R2 und R2' gleich oder verschieden sind und für Wasserstoff, C₁-C₁₈-Alkyl, Trifluormethyl, C₁-C₁₈-Alkylcarbonyl, C₅-C₆-Cycloalkyl oder Phenyl, welches unsubstituiert oder mit ein oder mehreren, z.B. 1, 2, 3 oder 4, Halogenatomen, Nitrogruppen, Trifluormethyl-, C₁-C₁₈-Alkyl-, C₁-C₁₈-Alkoxy-, C₁-C₁₈-Alkylcarbonyl- und/oder C₁-C₁₈-Alkoxycarbonyl-Gruppen substituiert sein kann, stehen.

Im festen Zustand kann die Verbindung der Formel (1) auch in einer anderen tautomeren, isomeren, oder tautomeren isomeren Form vorliegen.

Unter Mischkristallen werden im Sinne der vorliegenden Erfindung auch "feste Lösungen" oder "solid solutions" verstanden. Die Eigenschaften der Mischkristalle unterscheiden sich sowohl von den Eigenschaften der Einzelkomponenten, als auch von den Eigenschaften der physikalischen Mischungen der Einzelkomponenten. Insbesondere unterscheiden sich die Röntgenpulverdiagramme der Mischkristalle von der Summe der Pulverdiagramme der Einzelverbindungen.

Die Anteile der Einzelkomponenten in den erfindungsgemäßen Mischkristallen betragen für jede Einzelkomponente zwischen 10 und 90 Mol-%, insbesondere zwischen 20 und 60 Mol-%.
Bevorzugte binäre Mischkristalle bestehen aus zwei verschiedenen Verbindungen der Formel (1) im molaren Mengenverhältnis 1:3 bis 3:1.

Bevorzugte Verbindungen der Formel (1) sind solche, worin X und X' Wasserstoff, Fluor, Chlor oder Brom bedeuten.
Bevorzugte Verbindungen der Formel (1) sind weiterhin solche, worin R1, R1', R2 und R2' für Wasserstoff, C₁-C₄-Alkyl, insbesondere Methyl, Ethyl und Propyl, Trifluormethyl, C₁-C₄-Alkylcarbonyl, Cyclohexyl oder Phenyl, das unsubstituiert oder durch 1, 2 oder 3 Reste aus der Gruppe Fluor, Chlor, Brom, Nitro, Methyl, Ethyl, Propyl, Methoxy, Ethoxy, Methylcarbonyl, Methoxycarbonyl und/oder Ethoxycarbonyl substituiert ist, stehen.

Besonders bevorzugte Verbindungen der Formel (1) sind solche, worin X=X'=Cl, R2=R2'=H, und R1 und R1' unabhängig voneinander Methyl oder Ethyl sind.

Beispiele für Benzimidazolon-Verbindungen der allgemeinen Formel (1), die bevorzugte Mischkristalle bilden, sind die Verbindungen der folgenden Formeln: 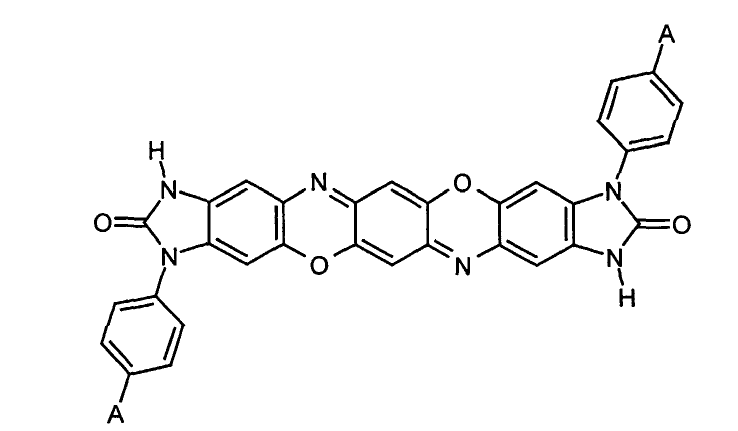
(1a): A=H
(1b): A=Cl
(1c): A=CH₃

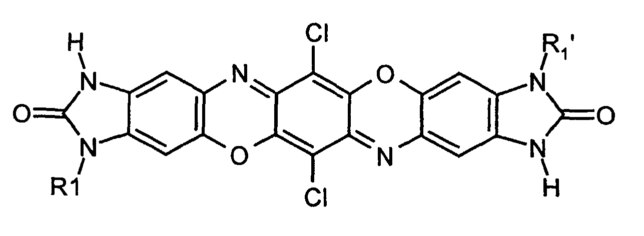

(1m): R1=R1'=CH₃
(1e): R1=R1'=C₂H₅
(1me): R1=CH₃
(1u): R1=R1'=H
(1ue): R1=H, R1'=C₂H₅

Bevorzugte binäre Mischkristalle sind solche, worin die eine Komponente eine Verbindung der Formel (1a), (1b), (1c), (1m), (1e), (1me), (1u) oder (1ue), und die zweite Komponente eine andere aus diesen Formeln ausgewählte Verbindung ist.

Bevorzugte ternäre Mischkristalle sind solche, worin die eine Komponente eine Verbindung der Formel (1a), (1b), (1c), (1m), (1e), (1me), (1u) oder (1ue), und die beiden anderen Komponente jeweils eine andere aus diesen Formeln ausgewählte Verbindung ist.

Besonders bevorzugte binäre Mischkristalle sind solche aus den Verbindungen der Formel (1m) und (1e), insbesondere im molaren Verhältnis von 1:9 bis 9:1, insbesondere von 1:3 bis 3:1.

Besonders bevorzugte ternäre Mischkristalle sind solche aus den Verbindungen der Formel (1m), (1e) und (1me). Vorzugsweise liegt das molare Verhältnis zwischen (1m) und (1e) zwischen 1:10 und 10:1, insbesondere zwischen 1:3 und 3:1, und das molare Verhältnis von (1me) zur Summe der Molanteile von (1e) und (1m) zwischen 1:10 und 10:1, insbesondere zwischen 1:3 und 3:1.

Die erfindungsgemäßen Mischkristalle zeichnen sich durch rote bis blaue Farbtöne, hohe Farbstärken, gute Dispergierbarkeiten und gute Licht- und Wetterechtheiten aus, insbesondere auch durch unerwartete Farbtöne. Beispielsweise ist ein ternärer Mischkristall aus den Komponenten (1m), (1e) und (1me) in Alkyd-Melamin-Lack nicht nur deutlich röter als eine physikalische Mischung aus den drei Einzelkomponenten (1m), (1e) und (1me), sondern auch deutlich röter als jede der unter denselben Bedingungen hergestellten Einzelkomponenten (1m), (1e) und (1me), obwohl (1m) rotviolett, (1e) hingegen blauviolett ist. Dies zeigt den großen Einfluss, den die Mischkristallbildung auf die Eigenschaften dieser Pigmente hat.

Die erfindungsgemäßen Mischkristalle können beispielsweise durch Cosynthese oder durch gemeinsames Umkristallisieren der verschiedenen Einzelverbindungen hergestellt werden.

Gegenstand der Erfindung ist auch ein Verfahren zur Herstellung der erfindungsgemäßen Mischkristalle, dadurch gekennzeichnet, dass man ein Gemisch aus zwei oder mehreren voneinander verschiedenen Verbindungen der Formel (2) 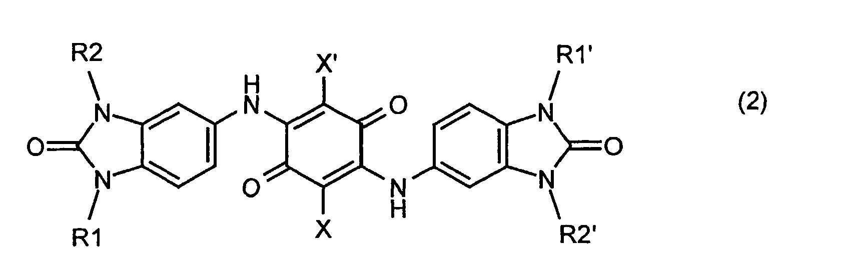 mit Schwefelsäure in Gegenwart eines Oxidationsmittels umsetzt,
oder indem man ein Gemisch aus zwei oder mehreren voneinander verschiedenen Verbindungen der Formel (3) 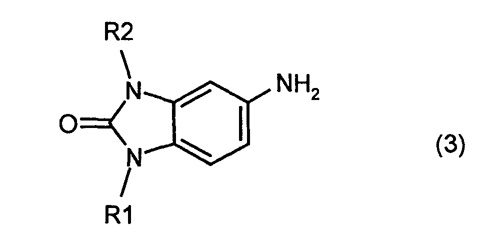 mit Chloranil umsetzt, und das Reaktionsprodukt mit Schwefelsäure in Gegenwart eines Oxidationsmittels umsetzt. Die Umsetzung mit Schwefelsäure kann bei Temperaturen zwischen -10°C und +50°C, bevorzugt zwischen 0 und 30°C, erfolgen. Die Menge an Mangandioxid kann 2 bis 4 Mol pro Mol der Verbindung der Formel (2) betragen.

Binäre Mischkristalle erhält man beispielsweise, wenn man ein Gemisch aus zwei verschiedenen Verbindungen der allgemeinen Formel (2) in Schwefelsäure mit einem Oxidationsmittel, z.B. Mangandioxid, umsetzt, wie vorstehend beschrieben.

Ternäre Mischkristalle erhält man beispielsweise, wenn man ein Gemisch aus zwei verschiedenen Verbindungen der allgemeinen Formel (3) mit Chloranil umsetzt und das entstandene Gemisch aus drei verschiedenen Verbindungen der Formel (2) in Schwefelsäure mit einem Oxidationsmittel, z.B. Mangandioxid, umsetzt. Als Lösemittel für die Umsetzung mit Chloranil kommen beispielsweise Ethanol, o-Dichlorbenzol, Dimethylformamid, Dimethylacetamid oder N-Methylpyrrolidon in Betracht. Die Umsetzung kann bei Temperaturen von 0 bis 250°C, vorzugsweise 20 bis 210°C, stattfinden. Die molare Menge an Chloranil zur Summe der Verbindungen der Formel (3) beträgt zweckmäßig 0,4 bis 0,6 zu 1.

Mischkristalle lassen sich auch herstellen, indem man ein Gemisch aus zwei oder mehreren, insbesondere zwei oder drei, verschiedenen Verbindungen der Formel (1) in Schwefelsäure, Dichloressigsäure und/oder Trifluoressigsäure auflöst und die Lösung auf Wasser oder Essigsäure gibt, und dadurch das Pigment als Mischkristall ausfällt. Im Falle von Trifluoressigsäure kann man auch die Trifluoressigsäure verdampfen, und dadurch das Pigment als Mischkristall ausfällen.

Darüber hinaus ist es auch möglich, durch gemeinsame Sublimation von zwei oder mehreren, insbesondere zwei oder drei, verschiedenen Benzimidazolondioxazinen der Formel (1) Mischkristalle herzustellen.

Die erfindungsgemäßen Mischkristalle können in verschiedenen polymorphen Formen vorliegen. Beispielsweise erhält man bei der Cosynthese von (1m), (1e) und (1me) einen ternären Mischkristall (1m/1e/1me) in der α-Phase, der sich bei Behandlung mit bestimmten Lösemitteln in einen anderen ternären Mischkristall mit derselben Zusammensetzung, aber einer anderen Kristallstruktur (β-Phase) umwandelt.

Drei unterschiedliche Modifikationen eines Mischkristalles (1a/1b) erhält man beispielsweise beim Umkristallisieren eines Gemisches von (1a) und (1b), je nachdem, ob man aus Schwefelsäure/Wasser oder aus Trifluoressigsäure/Wasser umkristallisiert, oder aus Trifluoressigsäure eindampft.

Die Bestimmung der Kristallmodifikation erfolgt durch Röntgenpulverdiffraktometrie.

In Abhängigkeit von der Reinheit der Edukte, den Konzentrationen, den angewandten Temperaturen und Temperaturverläufen, dem zeitlichen Verlauf der Synthese und einer eventuellen Nachbehandlung, dem Druck, der Anwesenheit von Verunreinigungen oder Additiven, und der Gegenwart von Impfkristallen können entweder nur Mischkristalle einer einzigen Phase, oder Mischkristalle verschiedener Phasen, oder eine Mischung aus Mischkristallen und einer oder mehreren Reinverbindungen entstehen.

Je nach gewünschtem Anwendungsbereich kann es sinnvoll sein, das erhaltene Pigment einer mechanischen Feinverteilung zu unterwerfen. Die Feinverteilung kann durch Nass- oder Trockenmahlung oder Kneten erfolgen. An die Mahlung bzw. Knetung kann sich eine Behandlung mit einem Lösemittel, mit Wasser, oder einem Lösemittel/Wasser-Gemisch anschließen.

Zur Erleichterung der Mischkristallbildung, zur Stabilisierung der Mischkristalle, zur Verbesserung der coloristischen Eigenschaften und zur Erzielung bestimmter coloristischer Effekte können an beliebigen Stellen des Verfahrens Pigmentdispergatoren, oberflächenaktive Mittel, Entschäumer, Extender oder andere Zuschlagstoffe zugesetzt werden. Es können auch Mischungen dieser Zusatzstoffe verwendet werden. Die Zugabe der Zusatzstoffe kann auf einmal oder in mehreren Portionen erfolgen. Die Zusatzstoffe können an jedem Punkt der Synthese oder der verschiedenen Nachbehandlungen, oder nach den Nachbehandlungen zugegeben werden. Der am besten geeignete Zeitpunkt muss zuvor durch orientierende Versuche ermittelt werden.

Die erfindungsgemäßen Mischkristalle, oder eine Mischung, die diese Mischkristalle enthält, eignen sich zum Pigmentieren von Lacken und Kunststoffen und zur Herstellung von Druckfarben und Pigmentpräparationen.

Die erfindungsgemäßen Mischkristalle, oder eine Mischung, die diese Mischkristalle enthält, sind geeignet als Farbmittel in elektrophotographischen Tonern und Entwicklern, wie z.B. Ein- oder Zweikomponentenpulvertonern (auch Ein- oder Zweikomponenten-Entwickler genannt), Magnettonern, Flüssigtonern, Latextonern, Polymerisationstonern sowie Spezialtonern.

Typische Tonerbindemittel sind Polymerisations-, Polyadditions- und Polykondensationsharze, wie Styrol-, Styrolacrylat-, Styrolbutadien-, Acrylat-, Polyester-, Phenol-Epoxidharze, Polysulfone, Polyurethane, einzeln oder in Kombination, sowie Polyethylen und Polypropylen, die noch weitere Inhaltsstoffe, wie Ladungssteuermittel, Wachse oder Fließhilfsmittel, enthalten können oder im Nachhinein mit diesen Zusätzen modifiziert werden.

Des weiteren sind die erfindungsgemäßen Mischkristalle, oder eine Mischung, die diese Mischkristalle enthält, geeignet als Farbmittel in Pulver und Pulverlacken, insbesondere in triboelektrisch oder elektrokinetisch versprühbaren Pulverlacken, die zur Oberflächenbeschichtung von Gegenständen aus beispielsweise Metall, Holz, Kunststoff, Glas, Keramik, Beton, Textilmaterial, Papier oder Kautschuk zur Anwendung kommen.

Als Pulverlackharze werden typischerweise Epoxidharze, carboxyl- und hydroxylgruppenhaltige Polyesterharze, Polyurethan- und Acrylharze zusammen mit üblichen Härtern eingesetzt. Auch Kombinationen von Harzen finden Verwendung. So werden beispielsweise häufig Epoxidharze in Kombination mit carboxyl- und hydroxylgruppenhaltigen Polyesterharzen eingesetzt. Typische Härterkomponenten (in Abhängigkeit vom Harzsystem) sind beispielsweise Säureanhydride, Imidazole sowie Dicyandiamid und deren Abkömmlinge, verkappte Isocyanate, Bisacylurethane, Phenol- und Melaminharze, Triglycidylisocyanurate, Oxazoline und Dicarbonsäuren.

Außerdem sind die erfindungsgemäßen Mischkristalle, oder eine Mischung, die diese Mischkristalle enthält, als Farbmittel in Tinten, vorzugsweise Ink-Jet Tinten, wie z.B. auf wässriger oder nichtwässriger Basis, Mikroemulsionstinten sowie in solchen Tinten, die nach dem Hot-melt-Verfahren arbeiten, geeignet. Ink-Jet-Tinten enthalten im allgemeinen insgesamt 0,5 bis 15 Gew.-%, vorzugsweise 1,5 bis 8 Gew.-%, (trocken gerechnet) einer oder mehrerer der erfindungsgemäßen Verbindungen.
Mikroemulsionstinten basieren auf organischen Lösemitteln, Wasser und ggf. einer zusätzlichen hydrotropen Substanz (Grenzflächenvermittler). Mikroemulsionstinten enthalten 0,5 bis 15 Gew.-%, vorzugsweise 1,5 bis 8 Gew.-%, einer oder mehrerer der erfindungsgemäßen Verbindungen, 5 bis 99 Gew.-% Wasser und 0,5 bis 94,5 Gew.-% organisches Lösungsmittel und/oder hydrotrope Verbindung.

"Solvent based" Ink-Jet-Tinten enthalten vorzugsweise 0,5 bis 15 Gew.-% einer oder mehrerer erfindungsgemäßer Verbindungen, 85 bis 99,5 Gew.-% organisches Lösungsmittel und/oder hydrotrope Verbindungen.

Hot-Melt-Tinten basieren meist auf Wachsen, Fettsäuren, Fettalkoholen oder Sulfonamiden, die bei Raumtemperatur fest sind und bei Erwärmen flüssig werden, wobei der bevorzugte Schmelzbereich zwischen ca. 60 und ca. 140°C liegt. Hot-Melt Ink-Jet-Tinten bestehen z.B. im wesentlichen aus 20 bis 90 Gew.-% Wachs und 1 bis 10 Gew.-% einer oder mehrerer der erfindungsgemäßen Verbindungen. Weiterhin können 0 bis 20 Gew.-% eines zusätzlichen Polymers (als "Farbstofflöser"), 0 bis 5 Gew.-% Dispergierhilfsmittel, 0 bis 20 Gew.-% Viskositätsveränderer, 0 bis 20 Gew.-% Plastifizierer, 0 bis 10 Gew.-% Klebrigkeitszusatz, 0 bis 10 Gew.-% Transparenzstabilisator (verhindert z.B. Kristallisation der Wachse) sowie 0 bis 2 Gew.-% Antioxidans enthalten sein. Typische Zusatzstoffe und Hilfsmittel sind z.B. in US-PS 5,560,760 beschrieben.

Außerdem sind die erfindungsgemäßen Mischkristalle, oder eine Mischung, die diese Mischkristalle enthält, auch geeignet als Farbmittel für Farbfilter, sowohl für die additive wie für die subtraktive Farberzeugung und für "elektronische Tinten".

Außerdem sind die erfindungsgemäßen Mischkristalle, oder eine Mischung, die diese Mischkristalle enthält, auch geeignet zur Einfärbung von Kosmetika, zur Papiermassefärbung und für Textildruck.

### Beispiele

In den folgenden Beispielen sind Teile und Prozentangaben auf das Gewicht bezogen. Der Nachweis der Mischkristalle und die Bestimmung der Kristallmodifikation der Mischkristalle erfolgt durch Röntgenpulverdiffraktometrie (Cu-K_{α}-Strahlung, doppelte Beugungswinkel 2Θ in Grad, in Klammern die Intensitäten). Die mit "breit" gekennzeichneten Linienlagen sind mit einer Ungenauigkeit von ± 0.4° behaftet, alle übrigen Linienlagen mit einer Ungenauigkeit von ± 0.2°.

### Beispiel 1: Cosynthese von (1m) und (1e)

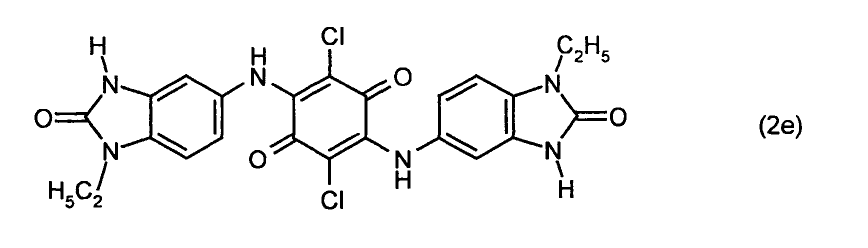

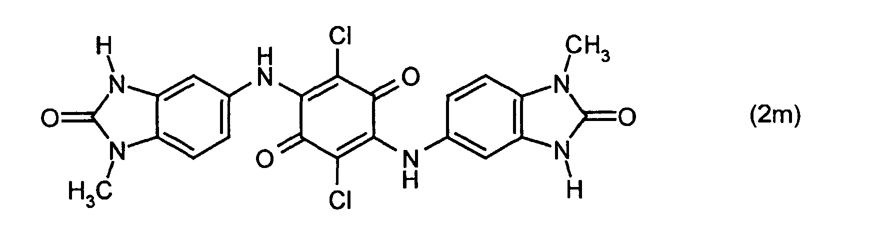

Ein Gemisch aus 79,2 Teilen der Verbindung (2e) und 75,3 Teilen der Verbindung (2m) wird bei einer Temperatur von 5 bis 15°C in 2760 Teile konzentrierte Schwefelsäure eingerührt. Man rührt noch 15 Minuten bei 5 bis 15°C nach, und gibt dann innerhalb von 5 Stunden 72 Teile aktiviertes Mangandioxid zu, wobei die Temperatur bei 5 bis 15°C gehalten wird. Der Überschuss an MnO₂ wird durch Zugabe von 15 Teilen 35%iger H₂O₂-Lösung zerstört. Man rührt noch 3 Stunden bei 5 bis 15°C nach und setzt anschließend so viel Wasser hinzu, dass die Schwefelsäure eine Konzentration von 80 % hat. Der ausgefallene Niederschlag wird abfiltriert, mit Schwefelsäure und Wasser gewaschen und getrocknet. Man erhält einen binären Mischkristall aus den Verbindungen (1m) und (1e), der folgende Linien im Röntgenpulverdiagramm zeigt (α'-Phase):
5,23 (schwach), 6,30 (schwach), 9,32 (mittel), 11,95 (schwach, breit), 19,93 (sehr schwach, breit), 26,78 (stark).

### Beispiel 2: Cosynthese von (1m), (1e) und (1me)

Zu einem Gemisch aus 750 Teilen Dimethylacetamid und 65,6 Teilen Natriumhydrogencarbonat gibt man ein Gemisch aus 65,2 Teilen 5-Amino-1-methyl-1,3-dihydrobenzimidazol-2-on (3m) und 70,8 Teilen 5-Amino-1-ethyl-1,3-dihydrobenzimidazol-2-on (3e). Man heizt die Reaktionsmischung auf 80°C und gibt innerhalb von viereinhalb Stunden 98,8 Teile Chloranil hinzu. Nach Beendigung der Reaktion wird die Mischung für eine Stunde zum Sieden erhitzt. Der Niederschlag wird heiß abfiltriert, gewaschen und getrocknet. Das so synthetisierte Gemisch aus Verbindungen der Formel (2) wird, wie Beispiel 1 beschrieben, mit Schwefelsäure/MnO₂ cyclisiert. Man erhält einen ternären Mischkristall aus den Verbindungen (1m), (1e) und (1me), der folgende Linien im Röntgenpulverdiagramm zeigt (α-Phase):
5,03 (mittel-schwach), 6,11 (schwach, breit), 10,02 (mittel-stark), 11,21 (sehr schwach, breit), 20,74 (sehr schwach, breit), 26,63 (stark).

### Beispiel 3: Phasenumwandlung eines binären Mischkristalles aus (1m) und (1e)

1 Teil des binären Mischkristalles aus Beispiel 1 wird in einer Mischung aus 1,5 Teilen Wasser und 8,5 Teilen Isobutanol suspendiert und 5 Stunden am Rückfluss erhitzt, abfiltriert, gewaschen und getrocknet. Man erhält eine neue Kristallmodifikation des binären Mischkristall aus den Verbindungen (1m) und (1e), die folgende Linien im Röntgenpulverdiagramm zeigt (β'-Phase):
5,09 (schwach), 6,36 (mittel), 10,78 (mittel-stark), 12,07 (schwach, breit) 14,17 (mittel-schwach), 19,37 (schwach), 20,75 (schwach), 21,69 (schwach), 23,07 (schwach), 24,88 (schwach), 25,97 (mittel), 26,94 (stark), 28,09 (sehr schwach)

### Beispiel 4: Phasenumwandlung eines ternären Mischkristalles aus (1m), (1e) und (1 me)

1 Teil des ternären Mischkristalles aus Beispiel 2 wird in 10 Teilen N-Methylpyrrolidon suspendiert und 3 Stunden bei 130°C erhitzt, abfiltriert, gewaschen und getrocknet.
Man erhält eine neue Kristallmodifikation des ternären Mischkristalls aus den Verbindungen (1m), (1e) und (1me), die folgende Linien im Röntgenpulverdiagramm zeigt (β-Phase):
6,37 (mittel), 10,78 (mittel), 10,90 (mittel-stark), 13,91 (schwach), 14,39 (mittel-schwach), 19,48 (mittel-schwach), 20,71 (schwach), 21,82 (schwach), 23,07 (schwach), 24,74 (sehr schwach), 25,92 (mittel-schwach), 27,05 (stark), 28,15 (sehr schwach), 28,99 (schwach) .

### Beispiel 5: Phasenumwandlung eines ternären Mischkristalles aus (1m), (1e) und (1me)

1 Teil des ternären Mischkristalles aus Beispiel 2 wird in einer Mischung aus 1,5 Teilen Wasser und 8,5 Teilen Isobutanol suspendiert und 5 Stunden am Rückfluss gekocht, abfiltriert, gewaschen und getrocknet. Man erhält einen ternären Mischkristall aus den Verbindungen (1m), (1e) und (1me). Wie das Röntgenpulverdiagramm zeigt, hat der Mischkristall dieselbe Kristallmodifikation wie in Beispiel 4.

### Beispiel 6: Cosynthese von (1m), (1e) und (1me)

Man verfährt wie in Beispiel 2, setzt jedoch ein Gemisch aus 13 Teilen der Verbindung (3m) und 127,4 Teilen der Verbindung (3e) ein. 1 Teil des so hergestellten Pulvers wird in einer Mischung aus 1,5 Teilen Wasser und 8,5 Teilen Isobutanol suspendiert und 5 Stunden am Rückfluss gekocht, abfiltriert, gewaschen und getrocknet. Man erhält einen ternären Mischkristall aus den Verbindungen (1m), (1e) und (1me), der folgende Linien im Röntgenpulverdiagramm zeigt:
7,51 (mittel), 9.79 (stark), 10,78 (mittel-schwach), 13,13 (schwach), 13,79 (mittel), 15,97 (mittel-schwach), 19,32 (mittel), 20,08 (sehr schwach), 21,65 (mittel), 22,76 (mittel-schwach, breit), 24,45 (mittel-schwach), 26,17 (mittel), 26,67 (stark), 28,26 (mittel-schwach)

### Beispiel 7: Cosynthese von (1m), (1e) und (1me)

Man verfährt wie in Beispiel 2, setzt jedoch ein Gemisch aus 117,4 Teilen der Verbindung (3m) und 14,2 Teilen der Verbindung (3e) ein. 1 Teil des so hergestellten Pulvers wird in einer Mischung aus 1,5 Teilen Wasser und 8,5 Teilen Isobutanol suspendiert und 5 Stunden am Rückfluss gekocht, abfiltriert, gewaschen und getrocknet. Man erhält einen ternären Mischkristall aus den Verbindungen (1m), (1e) und (1me), der folgende Linien im Röntgenpulverdiagramm zeigt:
5,04 (schwach), 6,34 (mittel-schwach), 10,91 (mittel-schwach), 12,30 (mittel), 14,32 (schwach, breit), 16,80 (sehr schwach, breit), 19,91 (sehr schwach, breit), 23.42 (sehr schwach, breit), 25,10 (sehr schwach, breit), 26,98 (stark), 31,93 (sehr schwach, breit)

### Beispiel 8: Cosynthese von (1u), (1e) und (1ue)

Man verfährt wie in Beispiel 2, setzt jedoch ein Gemisch aus 70,8 Teilen der Verbindung (3e) und 59,6 Teilen 5-Amino-1,3-dihydrobenzimidazol-2-on (3u) ein. Man erhält einen ternären Mischkristall aus den Verbindungen (1e), (1u) und (1ue), der folgende Linien im Röntgenpulverdiagramm zeigt:
6,56 (mittel), 10,10 (stark), 13,28 (mittel), 20,14 (schwach, breit), 22,66 (schwach), 26,50 (mittel-stark), 27,16 (stark).

### Beispiel 9: Umkristallisieren von (1m) und (1e) in Schwefelsäure/Wasser

1,75 Teile Pigment der Formel (1m) und 1,75 Teile des Pigmentes der Formel (1e) werden in 92 Teilen Schwefelsäure gelöst und bei Raumtemperatur in 400 Teile Wasser gegeben. Die Mischung wird für 5 Minuten zum Sieden erhitzt, der Niederschlag wird abfiltriert, gewaschen und getrocknet. Man erhält eine Mischung aus zwei binären Mischkristallen, die jeweils aus (1m) und (1e) bestehen und den Mischkristallen aus Beispiel 1 und 3 entsprechen.

### Beispiel 10: Umkristallisieren von (1m) und (1e) in Schwefelsäure/Eisessig

Man verfährt wie im vorigen Beispiel, verwendet aber Essigsäure statt Wasser. Man erhält eine Mischung aus zwei binären Mischkristallen, die jeweils aus (1m) und (1e) bestehen und den Mischkristallen aus Beispiel 1 und 3 entsprechen.

### Beispiel 11: Umkristallisieren von (1a) und (1b) in Schwefelsäure/Wasser

Eine Mischung aus 0,25 Teilen Pigment der Formel (1a) und 0,25 Teilen des Pigmentes der Formel (1b) sowie 36,4 Teilen Schwefelsäure wird auf 100°C erhitzt. Innerhalb von 10 Minuten gibt man 29 Teile Wasser hinzu. Nach dem Abkühlen auf Raumtemperatur wird der Niederschlag abfiltriert, gewaschen und getrocknet. Man erhält einen binären Mischkristall aus (1a) und (1b), der folgende Linien im Röntgenpulverdiagramm zeigt:
6,76 (stark), 11,63 (schwach), 12,73 (schwach), 13,18 (schwach), 13,56 (schwach), 16,45 (schwach), 16,94 (schwach), 17,20 (schwach), 17,87 (schwach), 23,26 (schwach), 24,09 (sehr schwach), 25,57 (mittel-schwach), 26,56 (mittel), 27,13 (mittel, breit)

### Beispiel 12: Umkristallisieren von (1a) und (1c) in Schwefelsäure/Wasser

Man verfährt wie im vorigen Beispiel, setzt jedoch 0,375 Teile des Pigmentes der Formel (1a) und 0,125 Teile Pigment der Formel (1c) ein. Man erhält einen binären Mischkristall aus (1a) und (1c), der folgende Linien im Röntgenpulverdiagramm zeigt:
6,68 (stark), 12,70 (schwach), 13,13 (schwach), 13,53 (schwach), 16,41 (schwach), 17,12 (schwach), 17,83 (sehr schwach), 25,54 (schwach), 26,57 (mittel), 27,10 (mittel, breit)

### Beispiel 13: Umkristallisieren von (1a) und (1b) in Trifluoressigsäure/Wasser

Eine Mischung aus 1 Teil Pigment der Formel (1a) und 1 Teil Pigment der Formel (1b) wird in 148 Teilen Trifluoressigsäure gelöst. Man fügt 100 Teile Wasser hinzu, und erhitzt zum Sieden. Der Niederschlag wird abfiltriert, gewaschen und getrocknet. Man erhält einen binären Mischkristall aus (1a) und (1b), der folgende Linien im Röntgenpulverdiagramm zeigt:
7,52 (stark), 12,43 (schwach), 13,66 (schwach, breit), 15,08 (schwach, breit), 19,65 (sehr schwach, breit), 20,54 (schwach, breit), 22,96 (schwach, breit), 26,87 (stark), 29,19 (schwach, breit). Der Mischkristall ist nicht isotyp mit den anderen beiden aus (1a) und (1b) hergestellten Mischkristallen aus Beispiel 11 und 14.

### Beispiel 14: Umkristallisieren von (1a) und (1b) in Trifluoressigsäure

Eine Mischung aus 0,5 Teilen Pigment der Formel (1a) und 0,5 Teilen des Pigmentes der Formel (1b) wird in 104 Teilen Trifluoressigsäure gelöst. Man verdampft die Trifluoressigsäure bei Raumtemperatur und erhält einen binären Mischkristall aus (1a) und (1b), der nicht isotyp zu den beiden anderen aus (1a) und (1b) hergestellten Mischkristallen aus Beispiel 11 und 13 ist. Das Röntgenpulverdiagramm zeigt folgende Linien:
6,43 (stark), 7,70 (mittel-schwach), 9,29 (mittel-schwach), 11,97 (mittel-schwach), 13,89 (schwach), 16,19 (mittel), 16,79 (mittel-stark), 17,53 (schwach), 18,05 (mittel-schwach), 23,09 (schwach), 23,85 (mittel-schwach), 25,88 (mittel-stark), 26,68 (mittel-stark), 27,63 (mittel-schwach).

### Beispiel 15: Sublimation von (1m) und (1e)

1,75 Teile Pigment der Formel (1m) und 1,75 des Pigmentes der Formel (1e) werden gemischt und in einem Röhrenofen bei einem Druck von 0,1 Pa und einer Temperatur von 450°C sublimiert. Man erhält einen binären Mischkristall aus (1m) und (1e), der sich durch folgende Linien im Röntgenpulverdiagramm auszeichnet: 10,39 (stark), 13,30 (mittel), 22,83 (schwach), 26,75 (mittel), 31,07 (sehr schwach).

## Patentansprüche

1. Mischkristall aus zwei oder mehreren voneinander verschiedenen Benzimidazolondioxazin-Verbindungen der allgemeinen Formel (1) 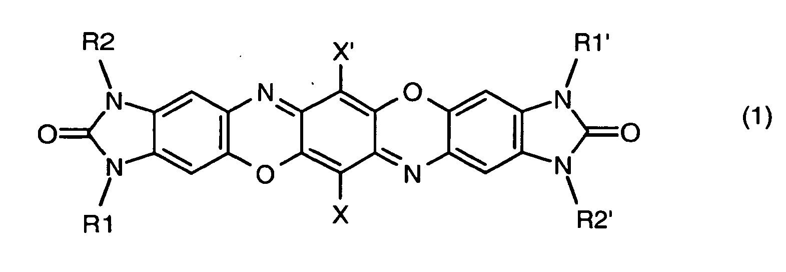 wobei
X und X' gleich sind und für Wasserstoff oder Halogen stehen, R1, R1', R2 und R2' gleich oder verschieden sind und für Wasserstoff, C₁-C₁₈-Alkyl, Trifluormethyl, C₁-C₁₈-Alkylcarbonyl, C₅-C₆-Cycloalkyl oder Phenyl, welches unsubstituiert oder mit ein oder mehreren Halogenatomen, Nitrogruppen, Trifluormethyl-, C₁-C₁₈-Alkyl-, C₁-C₁₈-Alkoxy-, C₁-C₁₈-Alkylcarbonyl- und/oder C₁-C₁₈₋Alkoxycarbonyl-Gruppen substituiert sein kann, stehen, und
**dadurch gekennzeichnet, dass** jede der voneinander verschiedenen Verbindungen der Formel (1) zu 10 bis 90 Mol-% im Mischkristall enthalten ist.

2. Mischkristall nach Anspruch 1, **dadurch gekennzeichnet, dass** jede der voneinander verschiedenen Verbindungen der Formel (1) zu 20 bis 60 Mol% im Mischkristall enthalten ist.

3. Mischkristall gemäß Anspruch 1oder 2, **dadurch gekennzeichnet, dass** X und X' Wasserstoff, Fluor, Chlor oder Brom bedeuten.

4. Mischkristall gemäß einem oder mehreren der Ansprüche 1 bis 3, **dadurch gekennzeichnet, dass** R1, R1', R2 und R2' für Wasserstoff, C₁-C₄-Alkyl, insbesondere Methyl, Ethyl und Propyl, Trifluormethyl, C₁-C₄-Alkylcarbonyl, Cyclohexyl oder Phenyl, das unsubstituiert oder durch 1, 2 oder 3 Reste aus der Gruppe Fluor, Chlor, Brom, Nitro, Methyl, Ethyl, Propyl, Methoxy, Ethoxy, Methylcarbonyl, Methoxycarbonyl und/oder Ethoxycarbonyl substituiert ist, stehen.

5. Mischkristall nach mindestens einem der Ansprüche 1 bis 4, **dadurch gekennzeichnet, dass** X=X'=CI, R2=R2'=H, und R1 und R1' unabhängig voneinander Methyl oder Ethyl sind.

6. Mischkristall nach mindestens einem der Ansprüche 1 bis 5, **dadurch gekennzeichnet, dass** der Mischkristall aus zwei verschiedenen Verbindungen der Formel (1) im molaren Mengenverhältnis 1:3 bis 3:1, besteht.

7. Mischkristall nach mindestens einem der Ansprüche 1 bis 6, **dadurch gekennzeichnet, dass** der Mischkristall aus den Verbindungen der Formel (1m) und (1e) besteht, vorzugsweise im molaren Verhältnis von 1:9 bis 9:1

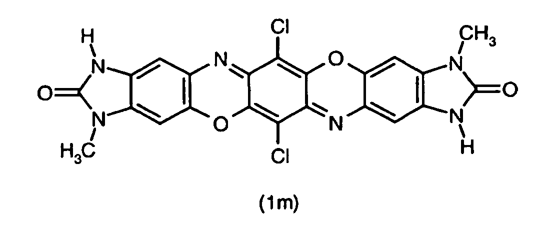

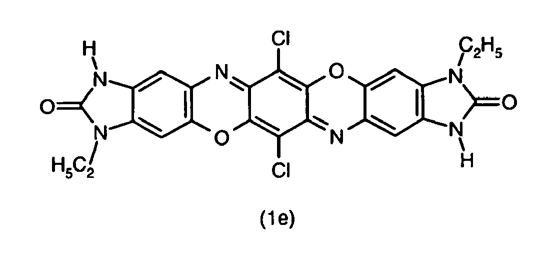

8. Mischkristall nach Anspruch 7, gekennzeichnet die folgende Linien im Röntgenpulverdiagramm (Cu-K_{α}-Strahlung, doppelte Beugungswinkel 2Θ in Grad, in Klammern die Intensitäten):
5,09 (schwach), 6,36 (mittel), 10,78 (mittel-stark), 12,07 (schwach, breit) 14,17 (mittel-schwach), 19,37 (schwach), 20,75 (schwach), 21,69 (schwach), 23,07 (schwach), 24,88 (schwach), 25,97 (mittel), 26,94 (stark), 28,09 (sehr schwach).

9. Mischkristall nach mindestens einem der Ansprüche 1 bis 5, **dadurch gekennzeichnet, dass** der Mischkristall aus den Verbindungen (1m), (1e) und (1 me) besteht

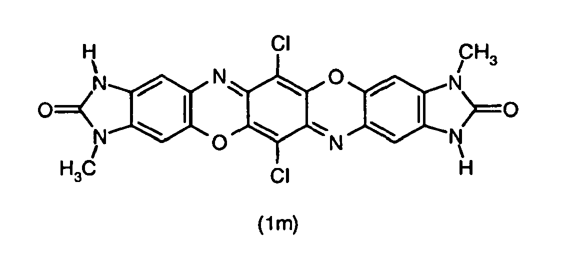

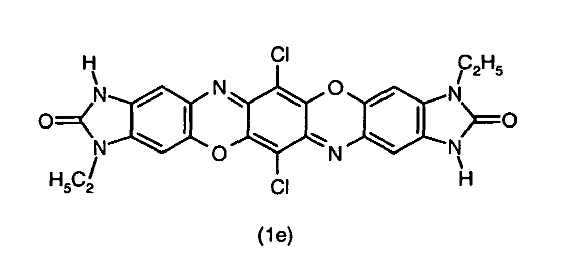

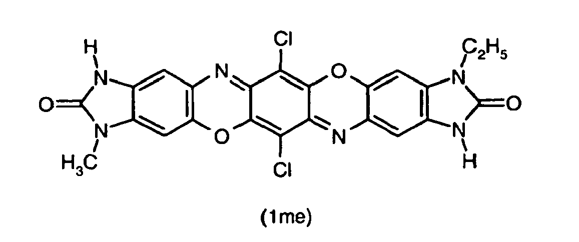

10. Mischkristall nach Anspruch 9, **dadurch gekennzeichnet, dass** das molare Verhältnis zwischen den Verbindungen der Formeln (1m) und (1e) zwischen 1:10 und 10:1, insbesondere zwischen 1:3 und 3:1, und das molare Verhältnis von (1me) zur Summe der Molanteile von (1e) und (1m) zwischen 1:10 und 10:1, insbesondere zwischen 1:3 und 3:1, liegt.

11. Mischkristall nach Anspruch 9 oder 10, **gekennzeichnet** die folgende Linien im Röntgenpulverdiagramm (Cu-K_{α}-Strahlung, doppelte Beugungswinkel 20 in Grad, in Klammern die Intensitäten):
6,37 (mittel), 10,78 (mittel), 10,90 (mittel-stark), 13,91 (schwach), 14,39 (mittel-schwach), 19,48 (mittel-schwach), 20,71 (schwach), 21,82 (schwach), 23,07 (schwach), 24,74 (sehr schwach), 25,92 (mittel-schwach), 27,05 (stark), 28,15 (sehr schwach), 28,99 (schwach).

12. Verfahren zur Herstellung von Mischkristallen gemäß einem oder mehreren der Ansprüche 1 bis 11, **dadurch gekennzeichnet, dass** man ein Gemisch aus zwei oder mehreren voneinander verschiedenen Verbindungen der Formel (2) 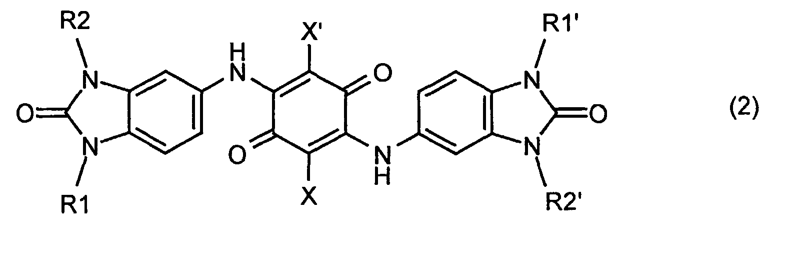 mit Schwefelsäure in Gegenwart eines Oxidationsmittels, vorzugsweise Mangandioxid, umsetzt, und gegebenenfalls mit polaren organischen Lösungsmitteln bei einer Temperatur zwischen 40 und 250°C behandelt.

13. Verfahren zur Herstellung von Mischkristallen gemäß einem oder mehreren der Ansprüche 1 bis 11, **dadurch gekennzeichnet, dass** man ein Gemisch aus zwei oder mehreren voneinander verschiedenen Verbindungen der Formel (3) 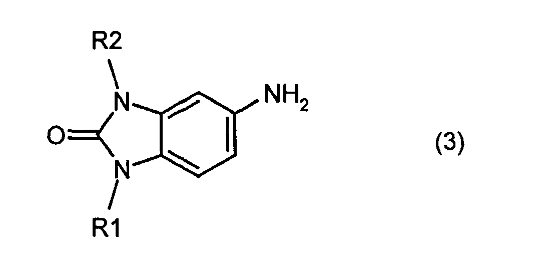 mit Chloranil umsetzt, und das entstandene Gemisch mit Schwefelsäure in Gegenwart eines Oxidationsmittels, vorzugsweise Mangandioxid, umsetzt, und gegebenenfalls mit polaren organischen Lösemitteln bei einer Temperatur zwischen 40 und 250°C behandelt.

14. Verfahren nach Anspruch 12 oder 13, **dadurch gekennzeichnet, dass** das polare organische Lösemittel ein C₁-C₂₀-Alkohol, vorzugsweise n-Butanol oder isoButanol, Dimethylformamid oder N-Methylpyrrolidon ist.

15. Verfahren zur Herstellung von Mischkristallen gemäß einem oder mehreren der Ansprüche 1 bis 11, **dadurch gekennzeichnet, dass** man ein Gemisch von mindestens zwei voneinander verschiedenen Verbindungen der Formel (1) in Schwefelsäure, Dichloressigsäure oder Trifluoressigsäure auflöst, und mit Wasser oder Essigsäure wieder ausfällt.

16. Verfahren zur Herstellung von Mischkristallen gemäß einem oder mehreren der Ansprüche 1 bis 11, **dadurch gekennzeichnet, dass** man ein Gemisch von zwei oder mehreren voneinander verschiedenen Verbindungen der Formel (1) gemeinsam sublimiert.

17. Verwendung der Mischkristalle gemäß einem oder mehreren der Ansprüche 1 bis 11 zum Pigmentieren von Lacken, Kunststoffen, Druckfarben, wässrigen oder lösemittelhaltigen Pigmentpräparationen, elektrophotographischen Tonern und Entwicklern, Pulverlacken, Tinten, vorzugsweise Ink-Jet-Tinten, Farbfiltern, und zum Einfärben von Saatgut und kosmetischen Artikeln, zur Papiermassefärbung und zum Textildruck.

## Claims

1. A mixed crystal of two or more different benzimidazolonedioxazine compounds of the formula (1) 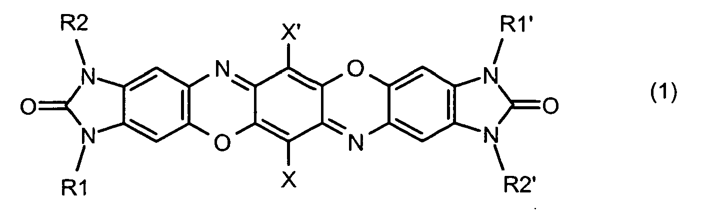 where
X and X' are identical and are hydrogen or halogen, R1, R1', R2 and R2' are identical or different and are hydrogen, C₁-C₁₈ alkyl, trifluoromethyl, C₁₋C₁₈ alkylcarbonyl, C₅-C₆ cycloalkyl or phenyl which may be unsubstituted or substituted by one or more halogen atoms, nitro groups, trifluoromethyl, C₁-C₁₈ alkyl, C₁-C₁₈ alkoxy, C₁-C₁₈ alkylcarbonyl and/or C₁-C₁₈ alkoxycarbonyl groups, and wherein each of the different compounds of the formula (1) is present in the mixed crystal at from 10 to 90 mol%.

2. The mixed crystal as claimed in claim 1, wherein each of the different compounds of the formula (I) is present in the mixed crystal at from 20 to 60 mol%.

3. The mixed crystal as claimed in claim 1 or 2, wherein X and X' are hydrogen, fluorine, chlorine or bromine.

4. The mixed crystal as claimed in one or more of claims 1 to 3, wherein R1, R1', R2 and R2' are hydrogen, C₁-C₄ alkyl, especially methyl, ethyl and propyl, trifluoromethyl, C₁-C₄ alkylcarbonyl, cyclohexyl or phenyl which is unsubstituted or substituted by 1, 2 or 3 radicals from the group consisting of fluorine, chlorine, bromine, nitro, methyl, ethyl, propyl, methoxy, ethoxy, methylcarbonyl, methoxycarbonyl and ethoxycarbonyl.

5. The mixed crystal as claimed in at least one of claims 1 to 4, wherein X=X'=Cl, R2=R2'=H, and R1 and R1' independently of one another are methyl or ethyl.

6. The mixed crystal as claimed in at least one of claims 1 to 5, consisting of two different compounds of the formula (1) in a molar ratio of from 1:3 to 3:1.

7. The mixed crystal as claimed in at least one of claims 1 to 6, consisting of the compounds of the formula (1 m) and (1e), preferably in a molar ratio of from 1:9 to 9:1

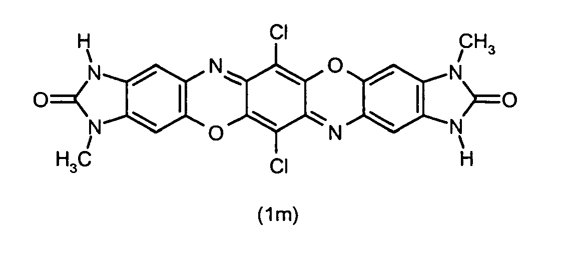

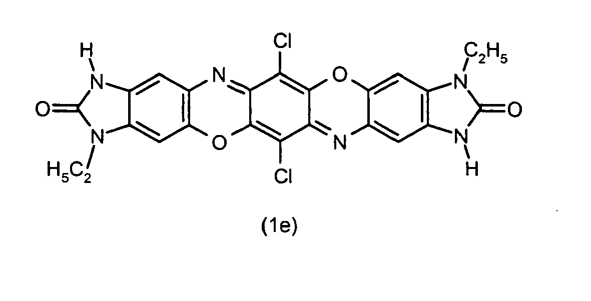

8. The mixed crystal as claimed in claim 7, **characterized by** the following lines in the X-ray powder diffraction pattern (Cu-K_{α} radiation, twice the Bragg angle 2Θ in degrees, the intensities in brackets).
5.09 (weak), 6.36 (moderate), 10.78 (moderate-strong), 12.07 (weak, broad), 14.17 (moderate-weak), 19.37 (weak), 20.75 (weak), 21.69 (weak), 23.07 (weak), 24.88 (weak), 25.97 (moderate), 26.94 (strong), 28.09 (very weak).

9. The mixed crystal as claimed in at least one of claims 1 to 5, consisting of the compounds (1m), (1e) and (1me)

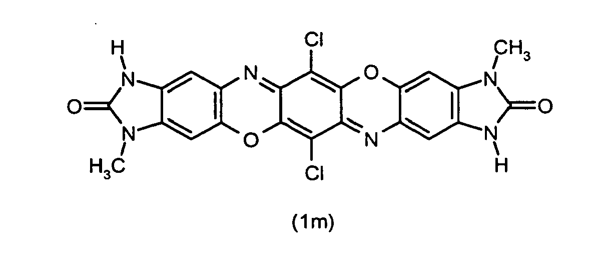

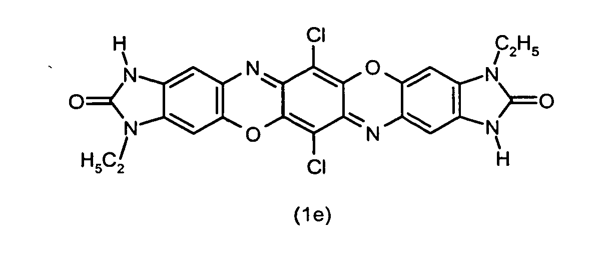

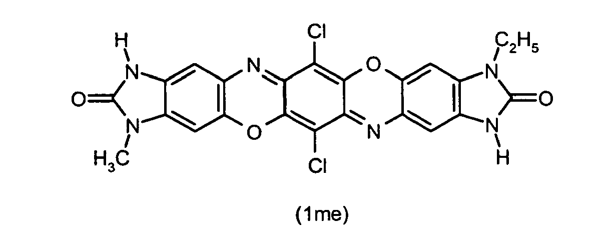

10. The mixed crystal as claimed in claim 9, wherein the molar ratio between the compounds of the formulae (1m) and (1e) is between 1:10 and 10:1, in particular between 1:3 and 3:1, and the molar ratio of (1me) to the sum of the molar fractions of (1e) and (1m) is between 1:10 and 10:1, in particular between 1:3 and 3:1.

11. The mixed crystal as claimed in claim 9 or 10, **characterized by** the following lines in the X-ray powder diffraction pattern (Cu-K_{α} radiation, twice the Bragg angle 2Θ in degrees, the intensities in brackets):
6.37 (moderate), 10.78 (moderate), 10.90 (moderate-strong), 13.91 (weak), 14.39 (moderate-weak), 19.48 (moderate-weak), 20.71 (weak), 21.82 (weak), 23.07 (weak), 24.74 (very weak), 25.92 (moderate-weak), 27.05 (strong), 28.15 (very weak), 28.99 (weak).

12. A process for preparing a mixed crystal as claimed in one or more of claims 1 to 11, which comprises reacting a mixture of two or more different compounds of the formula (2) 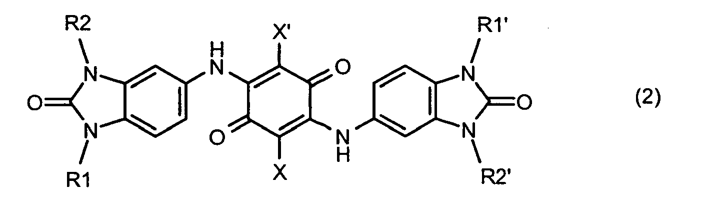 with sulfuric acid in the presence of an oxidizing agent, preferably manganese dioxide, and heating the product, where appropriate, with polar organic solvents at a temperature of between 40 and 250°C.

13. A process for preparing a mixed crystal as claimed in one or more of claims 1 to 11, which comprises reacting a mixture of two or more different compounds of the formula (3) 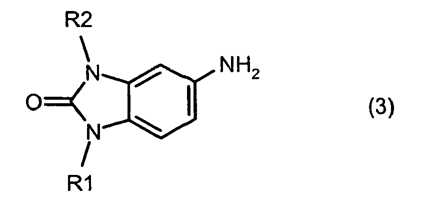 with chloranil, reacting the resulting mixture with sulfuric acid in the presence of an oxidizing agent, preferably manganese dioxide, and heating the product, where appropriate, with polar organic solvents at a temperature between 40 and 250°C.

14. The process as claimed in claim 12 or 13, wherein the polar organic solvent is a C₁-C₂₀ alcohol, preferably n-butanol or isobutanol, dimethylformamide or N-methylpyrrolidone.

15. A process for producing a mixed crystal as claimed in one or more of claims 1 to 11, which comprises dissolving a mixture of at least two different compounds of the formula (1) in sulfuric acid, dichloroacetic acid or trifluoroacetic acid and reprecipitating it with water or acetic acid.

16. A process for preparing a mixed crystal as claimed in one or more of claims 1 to 11, which comprises sublimating a mixture of two or more different compounds of the formula (1) together.

17. The use of a mixed crystal as claimed in one or more of claims 1 to 11, for pigmenting paints, plastics, printing inks, aqueous or solvent-based pigment preparations, electrophotographic toners or developers, powder coating materials, inks, preferably inkjet inks, color filters, or for coloring seed or cosmetic articles, for coloring paper in the mass, or for textile printing.

## Revendications

1. Cristal mixte constitué d'au moins deux benzimidazolonedioxazines différentes de formule générale (1) 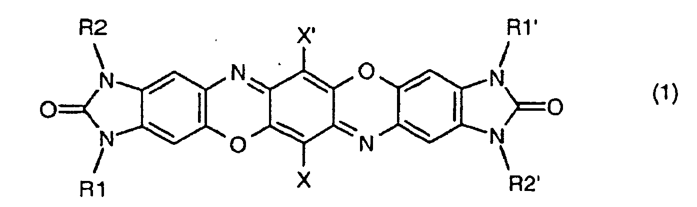 dans laquelle
X et X' sont identiques et représentent des atomes d'hydrogène ou d'halogène, R¹ R^{1'}, R² et R^{2'} sont identiques ou différents et représentent chacun un atome d'hydrogène, un groupe alkyle en C₁-C₁₈, trifluorométhyle, (alkyle en C₁-C₁₈) carbonyle, cycloalkyle en C₅-C₆ ou phényle, qui peut être non substitué, ou être substitué par un ou plusieurs atomes d'halogène, groupes nitro, groupes trifluorométhyle, alkyle en C₁-C₁₈, alcoxy en C₁₋C₁₈, (alkyle en C₁-C₁₈) carbonyle et/ou (alcoxy en C₁-C₁₈)-carbonyle, et
**caractérisé en ce que** chacun des composés différents les uns des autres de formule (1) est présent dans le cristal mixte en une quantité de 10 à 90 % en moles.

2. Cristal mixte selon la revendication 1, **caractérisé en ce que** chacun des composés différents les uns des autres de formule (1) est présent dans le cristal mixte en une quantité de 20 à 60 % en moles.

3. Cristal mixte selon la revendication 1 ou 2, **caractérisé en ce que** X et X' désignent des atomes d'hydrogène, de fluor, de chlore ou de brome.

4. Cristal mixte selon l'une ou plusieurs des revendications 1 à 3, **caractérisé en ce que** R¹, R¹ , R² et R^{2'} représentent chacun un atome d'hydrogène, un groupe alkyle en C₁-C₄, en particulier méthyle, éthyle et propyle, trifluorométhyle, (alkyle-en C₁-C₄)carbonyle, cyclohexyle ou phényle, qui est non substitué, ou est substitué par 1, 2 ou 3 radicaux choisis dans l'ensemble comprenant les groupes fluoro, chloro, bromo, nitro, méthyle, éthyle, propyle, méthoxy, éthoxy, méthylcarbonyle, méthoxycarbonyle et/ou éthoxycarbonyle.

5. Cristal mixte selon au moins l'une des revendications 1 à 4, **caractérisé en ce que** X = X' = Cl, R² = R^{2'} = H, et R¹ et R^{1'} représentent chacun indépendamment de l'autre un groupe méthyle ou éthyle.

6. Cristal mixte selon au moins l'une des revendications 1 à 5, **caractérisé en ce que** le cristal mixte est constitué de deux composés différents de formule (1) selon un rapport en moles de 1:3 à 3:1.

7. Cristal mixte selon au moins l'une des revendications 1 à 6, **caractérisé en ce que** le cristal mixte est constitué des composés de formules (1m) et (1e) selon un rapport en moles de 1:9 à 9:1.

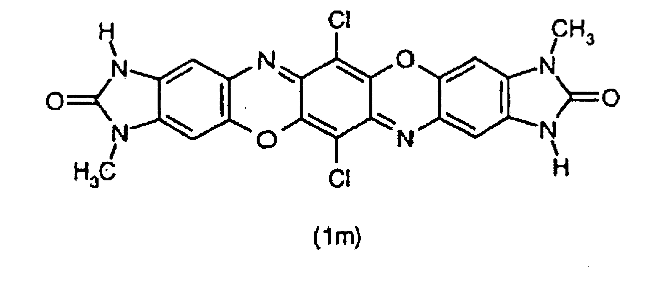

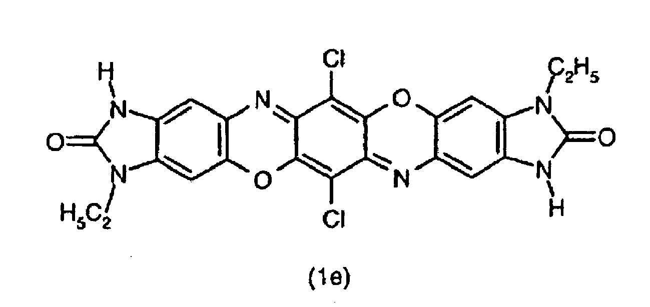

8. Cristal mixte selon la revendication 7, **caractérisé par** les raies suivantes dans l'image de diffraction aux rayons X par la méthode des poudres (radiation K_{α} de Cu, double de l'angle de diffraction 2θ en degrés, les intensités étant entre parenthèses) : 5,09 (faible), 6,36 (moyenne), 10,78 (moyenne-forte), 12,07 (faible, large), 14,17 (moyenne-faible), 19,37 (faible), 20,75 (faible), 21,69 (faible), 23,07 (faible), 24,88 (faible), 25,97 (moyenne), 26,94 (forte), 28,09 (très faible).

9. Cristal mixte selon au moins l'une des revendications 1 à 5, **caractérisé en ce que** le cristal mixte est constitué des composés (1m), (1e) et (1me).

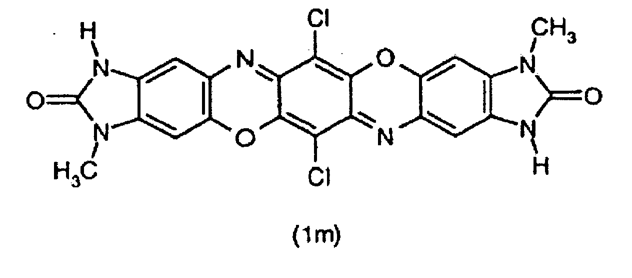

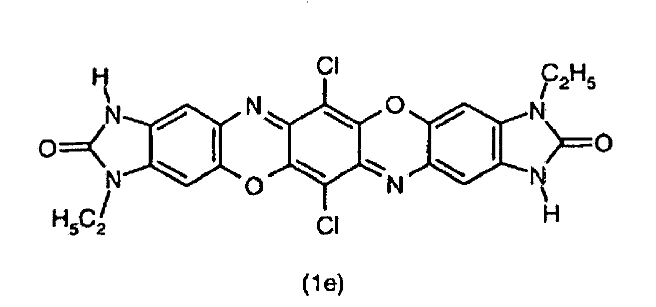

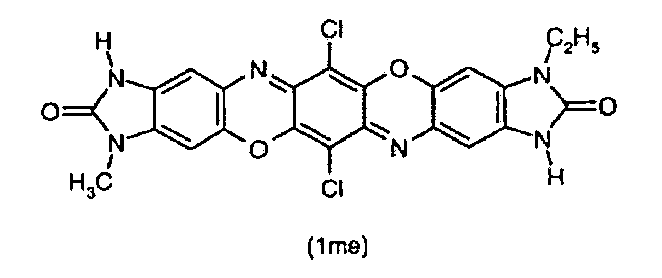

10. Cristal mixte selon la revendication 9, **caractérisé en ce que** le rapport en moles entre les composés de formules (1m) et (1e) est de 1:10 à 10:1, en particulier de 1:3 à 3:1, et que le rapport en moles de (1me) à la somme des parties en moles de (1e) et de (1m) est compris entre 1:10 et 10:1, en particulier entre 1:3 et 3:1.

11. Cristal mixte selon la revendication 9 ou 10, **caractérisé par** les raies suivantes dans l'image de diffraction aux rayons X par la méthode des poudres (radiation K_{α} de Cu, double de l'angle de diffraction 2θ en degrés, les intensités étant entre parenthèses) : 6,37 (moyenne), 10,78 (moyenne), 10,90 (moyenne-forte), 13,91 (faible), 14,39 (moyenne-faible), 19,48 (moyenne-faible), 20,71 (faible), 21,82 (faible), 23,07 (faible), 24,74 (très faible), 25,92 (moyenne-faible), 27,05 (forte), 28,15 (très faible), 28,99 (faible).

12. Procédé de préparation de cristaux mixtes selon l'une ou plusieurs des revendications 1 à 11, **caractérisé en ce qu'**on fait réagir un mélange d'au moins deux composés différents l'un de l'autre de formule (2) 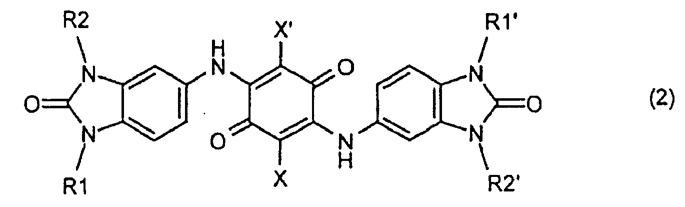 avec de l'acide sulfurique en présence d'un oxydant, de préférence le dioxyde de manganèse, et éventuellement on le traite avec des solvants organiques polaires à une température comprise entre 40 et 250°C.

13. Procédé de préparation de cristaux mixtes selon une ou plusieurs des revendications 1 à 11, **caractérisé en ce qu'**on fait réagir un mélange d'au moins deux composés différents l'un de l'autre de formule (3) 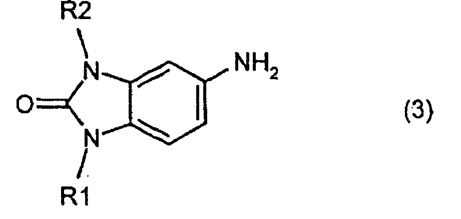 avec du chloranile, et on fait réagir le mélange ainsi obtenu avec de l'acide sulfurique en présence d'un oxydant, de préférence le dioxyde de manganèse, et éventuellement on le traite avec des solvants organiques polaires à une température comprise entre 40 et 250°C.

14. Procédé selon la revendication 12 ou 13, **caractérisé en ce que** le solvant organique polaire est un alcool en C₁-C₂₀, de préférence le n-butanol ou l'isobutanol, le diméthylformamide ou la N-méthylpyrrolidone.

15. Procédé de préparation de cristaux mixtes selon l'une ou plusieurs des revendications 1 à 11, **caractérisé en ce qu'**on dissout un mélange d'au moins deux composés différents l'un de l'autre de formule (1) dans de l'acide sulfurique, de l'acide dichloracétique ou de l'acide trichloracétique, et on le fait précipiter de nouveau avec de l'eau ou de l'acide acétique.

16. Procédé de préparation de cristaux mixtes selon l'une ou plusieurs des revendications 1 à 11, **caractérisé en ce qu'**on sublime ensemble un mélange d'au moins deux composés différents l'un de l'autre de formule (1).

17. Utilisation des cristaux mixtes selon l'une ou plusieurs des revendications 1 à 11 pour pigmenter des peintures et vernis, des plastiques, des encres d'imprimerie, des préparations pigmentaires aqueuses ou aux solvants, des toners et révélateurs électrophotographiques, des peintures en poudre, des encres, de préférence des encres pour impression au jet d'encre, des filtres colorés, et pour colorer dans la masse des semences et des objets à usage cosmétique, pour la coloration du papier dans la masse et pour l'impression textile.
